# EUROPEAN PATENT SPECIFICATION

(11) **EP 1 771 141 B1**
(45) Date of publication and mention of the grant of the patent: **25.03.2009**
(21) Application number: 05745642.8
(22) Date of filing: 13.05.2005
(51) Int. Cl.: A61K 6/00, A61K 6/08

(54) **DENTAL ADHESIVE COMPOSITION**
DENTALKLEBERZUSAMMENSETZUNG
COMPOSITION DENTAIRE ADHESIVE

(30) Priority: 27.07.2004 EP 04017749
(43) Date of publication of application: 11.04.2007
(73) Proprietor: DENTSPLY DETREY GmbH, 78467 Konstanz (DE)
(72) Inventor: BLACKWELL, Gordon, B., 78465 Konstanz (DE)
(74) Representative: Hartz, Nikolai
(86) International application number: PCT/EP2005/005263
(87) International publication number: WO 2006/010392

(56) References cited:
- EP-A- 0 351 076
- WO-A-03/013444

## Description

### Field of the Invention

The present invention relates to a photopolymerizable acidic one-component (one pack) dental adhesive composition containing acrylic esters and a specific solvent. Moreover, the present invention relates to the use of the specific solvent in a dental composition. The dental adhesive composition is characterized by a high stability during storage with regard to transesterification, solvent evaporation, and filler sedimentation as well as high resistance against deterioration of adhesive strength even after storage over an extended period of time.

### Background of the Invention

EP-A 0351 076 discloses dental adhesive compositions comprising a radical polymerizable monomer and an organic peroxide as essential components, which are dissolved in a volatile tertiary alcohol. Due to the low stability of organic peroxides in acidic media, the dental compositions can only have a limited acidity. Accordingly, acidic components are only present in an amount of about 1 weight % according to the examples of this reference.

WO03/013444 discloses a one part self-priming dental adhesive containing hydrolysis-stable polymerizable (meth)acrylamides.

Because the oral environment is always moist, dental adhesives require a polar solvent in order to be able to spread out on and wet the tooth surface. Water could be the ideal solvent from the polarity point of view, but many acrylate monomers are essentially insoluble in water. Also when acrylate monomers are stored in polar solvents capable of acting as nucleophiles such as water or ethanol the acrylate monomers are unstable and tend to either hydrolyze or trans-esterify so that a much decreased storage life is obtained. Since the hydrolysis and trans-esterification reactions are acid catalyzed, these reactions are even more of a problem when it is desired to store the solvent, acrylic monomer, and acid together in one container, as is normally the case with modern adhesives. Even when the adhesion values are only slightly affected, the reaction products such as ethyl acrylate have a strong and unpleasant odor making continued use impossible. Using a solvent such as acetone overcomes this last problem, but acetone readily passes through many common packaging materials such as polyethylene, and special packaging is therefore required which tends to be expensive. Water is easy to package has a relatively low volatility, and water is therefore more difficult to remove from the tooth surface than either ethanol or acetone. Ethanol does not easily pass through polyethylene packaging and volatizes more easily than water. However ethanol is not the preferred solvent for the resins because it can undergo reactions with the other components as described above.

Many attempts to prepare stable adhesive compositions for dental use have been based on ethanol as solvent, as in US 4,657,941 to Blackwell, and US 4,966,934 to Huang. These are intended as promoters of adhesion to dentin and describe formulations which are either applied alone or successively with further formulations. Adhesion to dentin up to 8 MPa is claimed in the forgoing patents, but there is no mention of adhesion to either etched or unetched enamel. In these patents ethanol is given as the preferred solvent although acetone and ethyl acetate are mentioned as possible alternatives. US 454,467 titled "Light-curable dentin and enamel adhesive" describes adhesive systems based an ethanol and containing a sulfur compound, and claims maximum adhesion to dentin of 8.4 MPa, and 19 MPa to etched enamel, US 5,089,051 to Eppinger describes a one component adhesive formulation comprising an acryloyloxy- or diacryloyloxy-alkyl dihydrogen phosphate together with an acidic carboxylic acid ester, and provides ethanol, propanol, isopropanol, butanol (normally understood to mean n-butanol), and acetone as suitable solvents. However, all examples use ethanol as solvent and the adhesion claimed to unetched enamel for the best example only is 8 MPa. Even with etched enamel a maximum adhesion of only 15 MPa was reached. Furthermore, the formulation does not comprise at least one acidic polymerisable adhesion promoter containing three or more acrylate groups. US 5,264,513 to Ikemura describes an adhesive formulation comprising water as solvent. The system requires the tooth to be treated with a separate primer and although adhesion to dentine up to 30 MPa was found, adhesion to unetched enamel was only 5.6 MPa. More recently, US 6,100,314 to Stefan describes aqueous formulations comprising various water soluble acrylic monomers and acidic monomers. However, with this system etching of the enamel and subsequent application of two different solutions is needed so that the present requirements of reduced time and effort on the part of the dentist is not fulfilled. In addition, maximum adhesion values of only 6.2 MPa are claimed to bovine enamel even after etching. Yamamoto in US 6,071,983 describes the same problems as outlined in the background to the present invention, and presents an adhesive system comprising a separate primer and adhesive. Water or ethanol are used as solvents. A special application tool is also described which can be pre-saturated with one component and thereby avoids having to mix the solution before use. However, the highest adhesion to dentin and enamel of 12.9 and 12.8 MPa respectively was found when the solutions were mixed in a dish just before use.

### Summary of the Invention

It is the problem of the present invention to provide a dental adhesive composition containing a solvent which is polar enough to allow good wetting of moist or polar surfaces, but which neither reacts with the components nor allows them to react with one another, is easy to remove by evaporation when required but does not pass easily through common packaging materials. Moreover, it is the problem of the invention to provide a dental adhesive composition having a high stability during storage with regard to transesterification, solvent evaporation, and filler sedimentation as well as high resistance against deterioration of adhesive strength even after storage over an extended period of time.

It is a further problem of the present invention to provide a dental adhesive composition which are stable, easily packaged, and which promote adhesion of dental radical polymerisable materials to both dentin and enamel with values over 18 MPa preferably without the need for prior acid etching or other treatment of the dentin or enamel.

These and other problems are solved according to claim 1 by a dental adhesive composition comprising a polymerisable monomer or prepolymer, and a solvent comprising t-butanol. The t-butanol is used in an amount effective for promoting the adhesion of the polymerisable monomer or prepolymer to hard body substances. The formulations of the present invention provide after one application adhesion to both unetched dentin and etched enamel of over 18 MPa. Formulations of the prior art therefore do not fulfill the requirements of the present invention. The present invention provides improved stability of adhesive formulations, together with easy packaging, both of which are advantageous to the dentist.

Accordingly, the present invention provides a one-component dental adhesive composition having a pH of at most 3, comprising
(a) a polymerisable acrylate monomer or prepolymer,
(b) a photoinitiator, and
(b) a solvent comprising t-butanol,
and which further contains a filler.

The present invent is based on the recognition that the dilemma between polarity and reactivity of the solvent of a dental adhesive composition may be overcome by the use of tertiary-butyl alcohol as the solvent. Tertiary-butyl alcohol is a solid at ambient temperature, and melts between about 25 and 26°C. It's use as a solvent for viscous acrylate monomers is therefore not obvious. However, it has been found that surprisingly formulations containing t-butanol can be obtained that are liquid at ambient temperatures when t-butanol is incorporated in a dental adhesive composition. The t-butyl alcohol is unable to hydrolyze or trans-esterify the monomers due to steric hindrance of the hydroxyl group by the methyl groups, is water miscible, and is polar enough to allow good wetting of moist or polar surfaces. t-Butyl alcohol vaporizes readily when this is required but does not readily pass through polyethylene packaging due to its polar hydroxyl group.

Moreover, the present invention is based on the recognition that a highly acidic one component dental adhesive composition with high storage stability, may be provided, which optionally contains a filler and which has a high acidity and a content of polymerisable acrylate monomer or prepolymer being unstable to hydrolysis and transesterification under strongly acidic conditions.

### Detailed description of the invention

The invention is described in more detail below. The term "acrylate" is used in this patent as a generic term to include acrylate, methacrylate, cyanoacrylate, and their derivatives. Also included are acrylic acid derivatives of the type disclosed in US 6,350,839. Specifically, the term "acrylate" relates to esters of acrylic acid, methacrylic acid and cyanoacrylic acid and their derivatives which are able to undergo esterification or transesterification reactions in ethanol at the acidic pH of the dental adhesive. Accordingly, a polymerisable acrylate monomer or prepolymer according to the present invention is an ester of acrylic acid, methacrylic acid and cyanoacrylic acid and their derivatives which is able to undergo transesterification reactions at room temperature in ethanol at the pH of the dental adhesive thereby forming ethyl esters having a strong and unpleasant smell. The term "acrylate" does not relate to hydrolysis-stable polymerizable (meth)acrylamides as disclosed in WO03/013444.

In the present invention compositions are provided that are useful for promoting the adhesion of dental materials to hard body substances such as tooth dentin, enamel, and bone. Use of these formulations allows preparation of the tooth surfaces for application of the polymerisable material in one application.

The dental adhesive composition is characterized by a high stability during storage with regard to transesterification, solvent evaporation, and filler sedimentation as well as high resistance against deterioration of adhesive strength even after storage over an extended period of time.

The dental adhesive composition according to the invention comprises a polymerisable acrylate monomer or prepolymer. The dental adhesive composition according to the invention may contain an acrylate polymerisable monomer or prepolymer as a mixture of different compounds or as isomers of the same compound. The polymerisable acrylate monomer or prepolymer may include a derivative of at least one unsaturated carboxylic acid selected from the group consisting of acrylic acid, methacrylic acid, cyanoacrylic acid and itaconic acid, and mixtures thereof. The polymerisable monomer or prepolymer may also include at least one unsaturated carboxylic acid selected from the group consisting of acrylic acid, methacrylic acid, cyanoacrylic acid and itaconic acid, and mixtures thereof. The polymerisable monomer or prepolymer may include a derivative of styrene, or a polymerisable moiety containing a carbon-carbon double bond conjugated with a carbonyl group.

The polymerisable acrylate monomer or prepolymer may also be selected from the group consisting of hydroxyethyl acrylate, hydroxypropyl acrylate, diurethane dimethacrylate resin, hydroxyethyl methacrylate, hydroxypropyl methacrylate, trimethylolpropane triacrylate, 1,6-hexanediacrylate, glycerin diacrylate, triethyleneglycol diacrylate, tetraethyleneglycol diacrylate, and a reaction product of butane tetracarboxylic acid dianhydride and hydroxyethylmethacrylate, triethyleneglycol dimethacrylate, urethane dimethacrylate, and a reaction product of butane tetracarboxylic acid dianhydride and glycerol dimethacrylate.

Additionally, the dental adhesive composition may further contain acrylamides or derivatives thereof such as 2-acrylamido-2-methylpropane sulphonic acid, N,N-methylene-bis-acrylamide, N,N-ethylene-bis-acrylamide, and 1,3-bis(acrylamido)-N,N-diethylpropane.

The polymerisable monomer or prepolymer may be a phosphate based acid adhesion promoter selected from the group consisting of phosphate ester or phosphonate derivatives of radical polymerisable alcohol or polyol derivatives. The phosphate ester derivatives may be prepared using the method given in US 4,514,342. As examples of suitable carboxylic acid based adhesion promoters may be mentioned the reaction product between butanetetracarboxylic acid dianhydride and hydroxylethyl acrylate as in US 5,218,070. Various radical polymerisable acidic monomers useful as adhesion promoters may also be obtained by many other means, for instance as given in US 4,806,381 and US 6,350,839.

The radical polymerisable alcohol or polyol derivative may be selected from the group consisting of hydroxyethyl acrylate, hydroxypropyl acrylate, glycerol diacrylate, pentaerythritol triacrylate, dipentaerythritol pentaacrylate, hexanediol acrylate, polyethylenoxide acrylate and triallylpentaerythritol.

The polymerisable monomer or prepolymer may further be a carboxylic acid based adhesion promoter selected from the group consisting of reaction products between acid anhydrides and radical polymerisable derivatives of alcohols. The acid anhydride may be selected from the group consisting of butanetetracarboxylic acid dianhydride, tetrahydrofurantetracarboxylic acid dianhydride, benzenetetracarboxylic acid dianhydride and benzentricarboxylic acid anhydride. The radical polymerisable derivatives of alcohols may be selected from the group consisting of hydroxyethyl acrylate, hydroxypropyl acrylate, glycerol diacrylate, pentaerythritol triacrylate, dipentaerythritol pentaacrylate, hexanediol acrylate, polyethylenoxide acrylate, and triallypentaerythritol.

These may be mono- or polyfunctional acrylates and methacrylates, of the kind described, for example, in EP-A-0 480 472. Moreover, functionalised monomers with terminal acrylate or methacrylate groups may likewise be used, of the kind described, e.g., in DE-A-2 312 559 and in EP-A-0 219 058.

The dental adhesive composition according to the invention further comprises a solvent comprising t-butanol. In a preferred embodiment, the dental adhesive composition contains t-butanol as the only solvent component. In a further preferred embodiment, the dental adhesive composition contains t-butanol and one or more further solvent components. The further solvent components may be selected from conventional inert solvents such as short-chain alcohols, short-chain ketones, aliphatic or unsaturated ethers, and cyclic ethers conventionally used in the dental field. The dental adhesive composition may contain the solvent in an amount of from 10 to 95 wt.%, whereby the solvent contains t-butanol in an amount of from 30 to 100 wt.-% effective for promoting the adhesion of the polymerisable monomer or prepolymer to hard body substances. Preferably, the dental composition contains 20 to 60 wt.%, in particular 20-30 or 40-60 wt.%,of t.-butanol as a solvent.

The dental adhesive composition according to the present invention may contain an acid. The acid may be an inorganic acid or an organic acid. Examples of the inorganic acid are conventional inorganic acids commonly used in the dental field such as phosphoric acid, sulfuric acid or hydrochloric acid. Examples of the organic acids are organic acids capable of taking part in a polymerisation reaction due to the presence of a polymerisable double bond in the molecule as described above, or conventional organic acids commonly used in the dental field such as tartraric acid, oxalic acid. Preferably, the dental adhesive composition according to the present invention has a pH of less than 3, preferably less than 2, most preferably a pH of less than 1.

The dental adhesive composition according to the present invention further comprised an initiator. In this case, the dental adhesive composition may be a one-component dental adhesive composition, wherein the initiator is a photoinitiator. The composition may comprise an alpha-diketone such as camphoroquinone.

The composition according to the present invention may also contain a filler, preferably a nanofiller, conventional stabilizers and auxiliaries. The filler may be contained in an amount of from 0.1 to 20 weight %, preferably 1 to 10 weight % based on the total weight of the composition. The particle size of nanofiller may be in a range of from 1 to 100 nm, preferably 2 to 20 nm. The average particle size may be in a range of from 0.5 to 50 nm, preferably 1 to 20 nm. Typical nanofillers may be Aerosils or Ormocers.

The composition according to the present invention may be packaged in a polyethylene container, as a one component formulation.

According to the present invention it is possible to provide a light-curable one-component dental adhesive composition having a highly acidic pH, which does not deteriorate over an extended period of time due to the reactivity of the ester group of acrylate monomers or prepolymers, sedimentation of an optional filler or evaporation of the solvent from the container.

Given that organic peroxides are unstable under the acidic conditions of the adhesive compositions of the invention, and given that any reducing agents, such as amines, used in combination with organic peroxides are ineffective under the acidic conditions of the adhesive compositions of the invention, an organic peroxide is not useful as an initiator according to the present invention. Moreover, given that the problem of esterification and transesterification does not arise with hydrolysis-stable acrylamides, the disclosure of hydrolysis-stable acrylamides represents an alternative approach to the stability problem of polymerizable monomers and prepolymers under acidic conditions. Finally, given the stabilization effect of the tert.-butanol with regard to the sedimentation problem of an optional filler, the present invention provides further advantages in the presence of a filler in the composition.

### EXAMPLES

Adhesion to dentin and enamel was tested as described below.

### Example 1

### Method for measurement of bond strength to tooth dentin and enamel

Human extracted teeth without significant anatomical alterations, defects or restorations were cleaned and disinfected by soaking in 1 % sodium hypochlorite solution for 18 to 24 hours, rinsed, and then stored at from 1 to 8°C in 1 % sodium chloride in water (saline solution) at about 4°C until used within six months.

Teeth were prepared for adhesion to dentin by abrading them using wet 300 grit silicon carbide paper to expose an area of dentin at a plane just below the original interface between the enamel and the dentin. This area was then polished by sanding with wet 600 grit silicon carbide paper. The teeth were kept in water until used within 1 to 12 hours.

When adhesion to enamel was to be tested, the teeth were chosen so that an approximately flat area of enamel about 5 mm in diameter was present. This area was washed and cleaned using wet 1200 grit silicon carbide powder and a bristle brush rotating in a dental hand piece. The teeth were then used within 6 hours as below.

The surface to be adhered to was dried lightly with a paper tissue. If etching was required the tooth surface was etched with either a commercially available etching agent (Etchgel, Dentsply) unless otherwise specified. The adhesive solution was then applied using a felt applicator tip as supplied by Dentsply International Inc. The adhesive solution was allowed to stand on the surface for 20 seconds and then the remaining solvent or water was evaporated by gently blowing with a stream of dry oil free air. The layer of remaining resin was light cured by irradiating it for ten seconds with light from a dental light curing unit having a minimum output of 350 milliwatt/square centimeter in the 400 to 500 nm wavelength range (For instance a Spectrum curing unit sold by Dentsply International Inc.). A portion of plastic straw of 5 mm internal diameter and about 4 mm long was placed end on to the Prepared surface and filled with a light curing dental filling material (Spectrum^{™} Dentsply International Inc.). Finally the filling material was cured by irradiating for forty seconds from the exposed end with the dental light.

The prepared samples were stored for 24 hours in water at 37°C before being tested in shear using a Zwick universal testing machine model Z01 0 with a cell having a maximum load of 500 Newtons, a crosshead speed of 1 mm per minute and a 2 mm diameter chisel. The chisel has a tip point formed at the lower end by grinding and polishing a planar surface across the end of the cylinder at a 45 degree angle to the central axis of the cylinder. In test position the tip point of the chisel was applied against the composite. Each tooth was mounted vertically in plaster for the test. A minimum of six samples were prepared for each test, and the mean adhesion was calculated. The invention is further illustrated by the examples given below. Abbreviations are used for the various resins, and the meaning and source of these is given first in the following table.

| | |
|---|---|
| Resin | meaning and source |
| PENTA | phosphate ester of pentaerythritol pentaacrylate, synthesized according to the method given in US4514342 |
| TCB | reaction product of butane tetracarboxylic acid dianhydride and hydroxyethylmethacrylate, prepared as in US5218070 |
| TGDMA | Triethyleneglycol dimethacrylate, obtained from Aldrich |
| UDMA | Urethane dimethacrylate, analogous to Plex 6661-0 available from Röhm, but with the hydroxyethyl groups replaced with hydroxypropyl groups |
| TCBG | Analogous to TCB but synthesized using glycerol dimethacrylate in place of hydroxyethyl methacrylate |

### Example 2

### Data showing comparative evaporation rates and improved packaging stability of the invention

The boiling points of various solvents, melting points, evaporation rate relative to ethanol, and dielectric constants of the pure solvents are presented in the next table. The boiling points, melting points and dielectric constants were obtained from the "CRC Handbook of Chemistry and Physics". The dielectric constant can be used as a guide to the suitability of a solvent for ionic reactions. The evaporation rates were determined by placing 1-3 grams of solvent into an aluminum dish on an electronic balance capable of weighing to 1 mg. Air was drawn across the balance pan at a velocity of approximately 20 m/min and the weight loss was determined at intervals of 30 seconds for five minutes. The evaporation rates in gm/second were thus obtained which allowed the evaporation rates relative to ethanol to be calculated. An evaporation rate similar to that of ethanol has been found to be advantageous.

| Polar solvent | b.p. °C | m.p.°C | evaporation rate relative to ethanol | dielectric constant |
|---|---|---|---|---|
| acetone | 56 | -94 | 3.06 | 20.7 |
| methanol | 64.7 | -98 | 1.10 | 32.6 |
| ethanol | 78 | -117 | 1.00 | 24.3 |
| n-propanol | 97 | -127 | 0.43 | 20.1 |
| iso-propanol | 82-84 | -89.5 | 0.78 | 18.3 |
| n-butanol | 117.7 | -90 | 0.17 | 17.8 |
| sec-butanol | 98 | -115 | 0.28 | 17.7 |
| t-butanol | 83 | 25-26 | 0.83 | 10.9 |
| water | 100 | 0 | 0.08 | 78.5 |

Ethanol is a preferred solvent from considerations of evaporation rate and dielectric constant, and it is well accepted as a solvent in dental formulations. However as explained earlier, ethanol reacts undesirably with many components of dental adhesives. Although methanol has a slightly higher rate of evaporation and higher dielectric constant it cannot be used for reasons of toxicity and reactivity. Acetone has good wetting properties but evaporates very quickly in an open dish and is difficult to package.

### Example 3

### Rate of loss of solvent from polyethylene bottles

Although many commercial adhesive systems for dentistry are now based on acetone, this is not an ideal solvent due to it's high volatility and ability to pass easily through the walls of polyethylene bottles. This necessitates either the use of glass bottles which many dentists don't like, or the use of plastic bottles incorporating a vapor barrier within the walls. An example of such a bottle is described in US6076709. However such multi-layer bottles tend to be expensive. The rate of loss of various solvents from normal polyethylene bottles was therefore determined as below. Polyethylene bottles with an internal volume of 4.5 ml and an average wall thickness of about 0.8 mm were partially filled with acetone, ethanol, iso-propanol, and t-butanol. The sealed bottles were weighed initially and then stored under ambient conditions. The bottles were re-weighed at suitable intervals and the average loss per day was calculated for each solvent. Results are given in the table below, and show that the loss of t-butanol through the walls of the bottles was approximately 1.5 times slower than iso-propanol, 2.5 times less than the loss of ethanol, and 20 times slower than the loss of acetone. Thus from the point of view of solvent loss from the packaging, t-butanol is highly preferred.

| Solvent | average weight loss per day through the walls of polyethylene bottles | relative rate (t-butanol= 1) |
|---|---|---|
| t-butanol | 0.4 mg/day | 1 |
| iso-propanol | 0.5 mg/day | 125 |
| ethanol | 1.0 mg/day | 25 |
| acetone | 8.0 mg/day | 20 |

### Example 4

### Storage stability of the solvent-acidic monomer mixtures

Three grams of a 30% solution of PENTA (a strongly acidic Phosphate and acrylate ester of dipentaerythritol) in ethanol were placed in a polyethylene bottle with a capacity of 4.5 ml and the top was closed tightly. The bottle was stored at 50°C to accelerate the aging process.

Three grams of a 30% solution of PENTA in iso-propanol were placed in a polyethylene bottle with a capacity of 4.5 ml and the top was closed tightly. The bottle was stored at 50°C to accelerate the aging process.

Three grams of a 30% solution of PENTA in t-butanol were placed in a polyethylene bottle with a capacity of 4.5 ml and the top was closed tightly. The bottle was stored at 50°C to accelerate the aging process.

After only twenty four hours the bottles containing the solution of PENTA in ethanol and iso-propanol smelled strongly and unpleasantly of acrylate esters. In contrast the smell of the bottle containing the solution of PENTA in t-butanol had not changed. After 5 days, vapor phase FTIR spectra were taken of the vapor above each liquid. All spectra showed absorptions due to the alcohols but the spectra of the vapor above the ethanol and iso-propanol solutions also showed absorptions at 1748 cm⁻¹ due to an ester C=O stretch. The absorbance of the peak at 1748 cm⁻¹ due to the ester formed in the various solutions is given in the table below. In the table the absorbance is corrected for differences in the baseline by subtracting the absorbance of the baseline at 1800 cm⁻¹. The characteristic infra-red absorption range for the C=O bond in esters is given as 1735 - 1750 cm⁻¹ in "Spectroscopic methods in Organic Chemistry" by Williams and Fleming (pub. McGraw-Hill).

| Alcohol | absorbance of the peak at 1748 cm⁻¹ due to ester group |
|---|---|
| ethanol | 2 |
| iso-propanol | 3 |
| t-butanol | not distinguishable from baseline |

This demonstrates that highest stability is obtained when t-butanol is used as solvent.

Compared to ethanol, the use of t-butanol as solvent therefore provides a lower rate of loss from polyethylene bottles, similar evaporation rate, and total lack of reaction towards the other constituents of the formulation, t-Butanol is therefore highly preferred over other solvents in formulations of the present invention.

### Example 5

### Stability of adhesive formulations with various solvents

Adhesive formulations were prepared by mixing together ingredients in the parts ratio given in the table below. Because these formulations are sensitive to yellow light, mixing was carried out in yellow light. When nano filler was used this was dispersed in the formulations by immersing a bottle containing the formulation in an ultra sound bath.

| Component | Example 1 | Example 2 | Example 3 | Example 4 | Example 5 | Example 6 | Example 7 |
|---|---|---|---|---|---|---|---|
| UDMA | 3.81 | 3.81 | 3.81 | 3.81 | 3.02 | 2.20 | 3.03 |
| PENTA | 23.14 | 23.14 | 23.14 | 23.14 | 18.35 | 17.4 | 18.44 |
| TGDMA | 3.84 | 3.84 | 3.84 | 3.84 | 3.04 | 2.20 | 3.06 |
| TCB | 1.32 | 7.32 | 7.32 | 7.32 | 5.82 | 9.60 | 5.84 |
| HEMA | 0.0 | 0.0 | 0.0 | 0.0 | 36.65 | | 36.83 |
| Methacrylic acid | | | | | | 3.30 | |
| t-Butanol | 60.09 | 60.09 | | | 24.5 | 62.3 | 24.5 |
| Ethanol | | | 60.09 | | | | |
| Acetone | | | | 60.09 | | | |
| Camphor quinone | 0.38 | 0.38 | 0.38 | 0.38 | 0.66 | 0.38 | 0.66 |
| DMABE* | 1.14 | 1.14 | 1.14 | 1.14 | 2.00 | 1.14 | 2.00 |
| BHT | .28 | 0.28 | 0.28 | 0.28 | 0.49 | 0.28 | |
| TBHQ** | | | | | | | 0.16 |
| nano filler | | 2.9 | 2.9 | 2.9 | 5.47 | | 5.47 |
| tooth etching required ? | 20% formic acid | yes | yes | yes | yes | no, self etching | yes |
| pH | 0 | 0 | 0 | 0 | 0 - 1 | 0 | 0 |

| | | | | | | | |
|---|---|---|---|---|---|---|---|
| *Dimethylaminobenzoic acid, ethyl ester ** t-butylhydroquinone. | | | | | | | |

The pH was measured using 0-6 pH indicator strips, article number 92115 from Machery-Nagel. The strips were lightly moistened with deionised water before being saturated with the adhesive solution to be measured. The lowest pH measurable with these indicators is 0.0.

The above example 2, 3, 4, and 5 were filled into polyethylene bottles as commonly used in the dental industry, and these were stored in an oven at 50°C. Samples were removed weekly or as appropriate and the stability was checked visually and by testing adhesion to dentine as described earlier. Results are given below, Examples 1 and 6 are provided merely as illustrations of further possible formulations.

### Example 6

### Adhesion to dentine in MPa after storage of the formulations at 50°C

| Days storage time | Example 1 | Example 2 | Example 3 | Example 4 | Example 5 | Example 6 | Example 7 |
|---|---|---|---|---|---|---|---|
| 0 | 19.9 (4) | 19.3 (4) | 23.6 (2) | 23.5 (3) | 19.2 (4.2) | 19.2 (4.1) | 23.6 (1.8) |
| 7 | 22.8 (3) | 20.5 (4) | | | 19.6 (2.6) | 19.1 (3.6) | |
| 14 | | | mixture smelled strongly of ethyl-acrylate | mixture was more viscous, and brown | | 20.4 (4.3) | |
| 21 | | | | mixture had polymerised | 17.7 (3.5) | 17.7 (3.2) | |
| 28 | 23.4 (4) | 20.7 (4) | 12.6 (2) | | | 20.9 (4.9) | 22.8 (2.4) |
| 56 | 18.3 (6) | 21.2 (4) | | | | | |
| 77 | 19.0(5) | | | | | | |
| 230 | 19.0 (5) | 19.0 (2) | | | | | |
| 366 | | 18.1 (4) | | | | | |

### Example 7

### Sedimentation of filler

The physical stability of the formulations containing nanofiller was tested by centrifuging these at the equivalent of 500 times normal gravity for 4 hours using a LumiFuge 114 test centrifuge.

### Results

| | |
|---|---|
| Example 2 | no sediment |
| Example 3 | light sediment |
| Example 4 | totally sedimented |

As a result it was found that the formulation containing t-butanol was physically more stable than those containing either ethanol or acetone.

## Claims

1. One component dental adhesive composition having a pH of at most 3, comprising
(a) a polymerisable acrylate monomer or prepolymer,
(b) a photoinitiator, and
(c) a solvent comprising t-butanol,
and which further contains a filler.

2. The dental adhesive composition according to claim 1, wherein the dental adhesive composition contains the solvent in an amount of from 10 to 95 wt.%, whereby the solvent contains t-butanol in an amount of from 30 to 100 wt.-% effective for promoting the adhesion of the polymerisable acrylate monomer or prepolymer to hard body substances.

3. The dental adhesive composition according to any one of the preceding claims wherein the polymerisable acrylate monomer or prepolymer includes a derivative of at least one unsaturated carboxylic acid selected from the group consisting of acrylic acid, methacrylic acid, cyanoacrylic acid and itaconic acid, and mixtures thereof.

4. The dental adhesive composition according to any one of the preceding claims wherein the polymerisable acrylate monomer or prepolymer is selected from the group consisting of hydroxyethyl acrylate, hydroxypropyl acrylate, diurethane dimethacrylate resin, hydroxyethyl methacrylate, hydroxypropyl methacrylate, trimethylolpropane triacrylate, 1,6-hexanediacrylate, glycerin diacrylate, triethyleneglycol diacrylate, and tetraethyleneglycol diacrylate, a reaction product of butane tetracarboxylic acid dianhydride and hydroxyethylmethacrylate, triethyleneglycol dimethacrylate, urethane dimethacrylate, and a reaction product of butane tetracarboxylic acid dianhydride and glycerol dimethacrylate.

5. The dental adhesive composition according to any one of the preceding claims wherein the polymerisable acrylate monomer or prepolymer is a phosphate based acid adhesion promoter selected from the group consisting of phosphate ester or phosphonate derivatives of radical polymerisable alcohol or polyol derivatives.

6. The dental adhesive composition according to claim 5 wherein the radical polymerisable alcohol or polyol derivative is selected from the group consisting of hydroxyethyl acrylate, hydroxypropyl acrylate, glycerol diacrylate, pentaerythritol triacrylate, dipentaerythritol pentaacrylate, hexanediol acrylate, and polyethylenoxide acrylate.

7. The dental adhesive composition according to any one of claims 1 to 4 wherein the polymerisable acrylate monomer or prepolymer is a carboxylic acid based adhesion promoter selected from the group consisting of reaction products between acid anhydrides and radical polymerisable acrylate derivatives of alcohols.

8. The dental adhesive composition according to claim 7 wherein the acid anhydride is selected from the group consisting of butanetetracarboxylic acid dianhydride, tetrahydrofurantetracarboxylic acid dianhydride, benzenetetracarboxylic acid dianhydride and benzentricarboxylic acid anhydride.

9. The dental adhesive composition according to claim 7 or 8 wherein the radical polymerisable derivatives of alcohols are selected from the group consisting of hydroxyethyl acrylate, hydroxypropyl acrylate, glycerol diacrylate, pentaerythritol triacrylate, dipentaerythritol pentaacrylate, hexanediol acrylate, polyethylenoxide acrylate, hydroxyethylmethacrylate, hydroxypropyl methacrylate, and glycerol dimethacrylate.

10. The dental adhesive composition according to any one of the preceding claims wherein the photoinitiator comprises an alpha-diketone.

11. The composition of claim 10, wherein said catalyst comprises camphorquinone.

12. The composition according to any one of the preceding claims, which is packaged in a polyethylene container.

13. The composition according to claim 1, wherein the filler is a nanofiller.

14. The composition according to any one of the preceding claims, wherein the adhesive composition has an adhesion to dentine of at least 18 MPa.

15. The composition according to any one of the preceding claims, which is a one component formulation.

16. The composition according to any one of the preceding claims, wherein the polymerisable monomer or prepolymer contains a functional group hydrolysable under acidic conditions in an aqueous medium.

## Patentansprüche

1. Einkomponentige Dentaladhäsivzusammensetzung mit einem pH-Wert von höchstens 3, die
(a) ein polymerisierbares Acrylatmonomer oder Prepolymer,
(b) einen Photoinitiator, und
(c) ein t-Butanol-enthaltendes Lösungsmittel,
umfasst, und die weiterhin einen Füllstoff enthält.

2. Die Dentaladhäsivzusammensetzung nach Anspruch 1, wobei die Dentaladhäsivzusammensetzung das Lösungsmittel in einer Menge von 10 bis 95 Gewichtsprozent enthält, wobei das Lösungsmittel t-Butanol in einer Menge von 30 bis 100 Gewichtsprozent enthält, die wirksam ist zur Verbesserung der Adhäsion des polymerisierbaren Acrylatmonomers oder Prepolymers an harte Körpersubstanz.

3. Die Dentaladhäsivzusammensetzung nach einem der vorstehenden Ansprüche, wobei das polymerisierbare Acrylatmonomer oder Prepolymer ein Derivat mindestens einer ungesättigten Carbonsäure enthält, die ausgewählt wird aus der Gruppe, bestehend aus Acrylsäure, Methacrylsäure, Cyanoacrylsäure und Itaconsäure, sowie Gemische davon.

4. Die Dentaladhäsivzusammensetzung nach einem der vorstehenden Ansprüche, wobei das polymerisierbare Acrylatmonomer oder Prepolymer ausgewählt wird aus der Gruppe, bestehend aus Hydroxyethylacrylat, Hydroxypropylacrylat, Diurethandimethacrylat-Harz, Hydroxyethylmethacrylat, Hydroxypropylmethacrylat, Trimethylolpropantriacrylat, 1,6-Hexandiacrylat, Glycerindiacrylat, Triethylenglycoldiacrylat, und Tetraethylenglycoldiacrylat, einem Reaktionsprodukt aus Butantetracarbonsäuredianhydrid und Hydroxyethylmethacrylat, Triethylenglycoldimethacrylat, Urethandimethacrylat, und einem Reaktionsprodukt aus Butantetracarbonsäuredianhydrid und Glycerindimethacrylat.

5. Die Dentaladhäsivzusammensetzung nach einem der vorstehenden Ansprüche, wobei das polymerisierbare Acrylatmonomer oder Prepolymer ein auf einem Phosphat basierender saurer Adhäsionspromoter ist, der ausgewählt wird aus der Gruppe bestehend aus Phosphatestern oder Phosphonatderivaten von radikalisch polymerisierbaren Alkohol- oder Polyolderivaten.

6. Die Dentaladhäsivzusammensetzung nach Anspruch 5, wobei das radikalisch polymerisierbare Alkohol oder Polyolderivat ausgewählt wird aus der Gruppe, bestehend aus Hydroxyethylacrylat, Hydroxypropylacrylat, Glycerindiacrylat, Pentaerythritholtriacrylat, Dipentaerythritholpentaacrylat, Hexandiolacrylat, und Polyethylenoxidacrylat.

7. Die Dentaladhäsivzusammensetzung nach einem der Ansprüche 1 bis 4, wobei das polymerisierbare Acrylatmonomer oder Prepolymer ein auf einer Carbonsäure beruhender Adhäsionsverstärker ist, der ausgewählt wird aus der Gruppe, bestehend aus Reaktionsprodukten zwischen Säureanhydriden und radikalisch polymerisierbaren Acrylatderivaten von Alkoholen.

8. Die Dentaladhäsivzusammensetzung gemäß Anspruch 7, wobei das Säureanhydrid ausgewählt wird aus der Gruppe, bestehend aus Butantetracarbonsäuredianhydrid, Tetrahydrofurantetracarbonsäuredianhydrid, Benzoltetracarbonsäuredianhydrid und Benzoltricarbonsäureanhydrid.

9. Die Dentaladhäsivzusammensetzung gemäß Anspruch 7 oder 8, wobei die radikalisch polymerisierbaren Alkoholderivate ausgewählt werden aus der Gruppe, bestehend aus Hydroxyethylacrylat, Hydroxypropylacrylat, Glycerindiacrylat, Pentaerythritoltriacrylat, Dipentaerythritolpentaacrylat, Hexandiolacrylat, Polyethylenoxidacrylat, Hydroxyethylmethacrylat, Hydroxypropylmethacrylat, und Glycerindimethacrylat.

10. Die Dentaladhäsivzusammensetzung gemäß einem der vorstehenden Ansprüche, wobei der Photoinitiator ein Alpha-Diketon umfasst.

11. Die Zusammensetzung nach Anspruch 10, wobei der Katalysator Kampferchinon umfasst.

12. Die Zusammensetzung nach einem der vorstehenden Ansprüche, die in einem Polyethylenbehälter verpackt ist.

13. Die Zusammensetzung nach Anspruch 1, wobei der Füllstoff ein Nanofüllstoff ist.

14. Die Zusammensetzung nach einem der vorstehenden Ansprüche, wobei die Adhäsivzusammensetzung eine Adhäsion an Dentin von mindestens 18 MPa aufweist.

15. Die Zusammensetzung nach einem der vorstehenden Ansprüche, die eine einkomponenten Formulierung ist.

16. Die Zusammensetzung nach einem der vorstehenden Ansprüche, wobei das polymerisierbare Monomer oder Prepolymer eine funktionelle Gruppe enthält, die unter sauren Bedingungen in einem wässrigen Medium hydrolysierbar ist.

## Revendications

1. Composition d'adhésif dentaire à un composant, ayant un pH d'au plus 3, comprenant
(a) un monomère ou prépolymère d'acrylate polymérisable,
(b) un photoinitiateur, et
(c) un solvant comprenant du tertiobutanol
et qui contient en outre une charge.

2. Composition d'adhésif dentaire suivant la revendication 1, ladite composition d'adhésif dentaire contenant le solvant en une quantité de 10 à 95 % en poids, ce solvant contenant du tertiobutanol en une quantité de 30 à 100 % en poids efficace pour activer l'adhérence du monomère ou prépolymère d'acrylate polymérisable à des substances consistant en corps durs.

3. Composition d'adhésif dentaire suivant l'une quelconque des revendications précédentes, dans laquelle le monomère ou prépolymère d'acrylate polymérisable comprend un dérivé d'au moins un acide carboxylique insaturé choisi dans le groupe consistant en l'acide acrylique, l'acide méthacrylique, l'acide cyanoacrylique, l'acide itaconique et leurs mélanges.

4. Composition d'adhésif dentaire suivant l'une quelconque des revendications précédentes, dans laquelle le monomère ou prépolymère d'acrylate polymérisable est choisi dans le groupe consistant en l'acrylate d'hydroxyéthyle, l'acrylate d'hydroxypropyle, une résine de diméthacrylate de diuréthanne, le méthacrylate d'hydroxyéthyle, le méthacrylate d'hydroxypropyle, le triacrylate de triméthylolpropane, le 1,6-hexanediacrylate, le diacrylate de glycérol, le diacrylate de triéthylèneglycol et le diacrylate de tétra-éthylèneglycol, un produit de réaction du dianhydride d'acide butanetétracarboxylique et du méthacrylate d'hydroxyéthyle, le diméthacrylate de triéthylèneglycol, le diméthacrylate d'uréthanne, et un produit de réaction du dianhydride d'acide butanetétracarboxylique et du diméthacrylate de glycérol.

5. Composition d'adhésif dentaire suivant l'une quelconque des revendications précédentes, dans laquelle le monomère ou prépolymère d'acrylate polymérisable est un activateur d'adhérence acide à base de phosphate choisi dans le groupe consistant en des dérivés consistant en esters du type phosphate ou phosphonates de dérivés d'alcools ou polyols polymérisables par radicaux.

6. Composition d'adhésif dentaire suivant la revendication 5, dans laquelle le dérivé d'alcool ou de polyol polymérisable par radicaux est choisi dans le groupe consistant en l'acrylate d'hydroxyéthyle, l'acrylate d'hydroxypropyle, le diacrylate de glycérol, le triacrylate de pentaérythritol, le pentaacrylate de dipentaérythritol, l'acrylate d'hexanediol et l'acrylate de poly(oxyde d'éthylène).

7. Composition d'adhésif dentaire suivant l'une quelconque des revendications 1 à 4, dans laquelle le monomère ou prépolymère d'acrylate polymérisable est un activateur d'adhérence à base d'acide carboxylique choisi dans le groupe consistant en les produits de réaction entre des anhydrides d'acides et des dérivés d'acrylates d'alcools, polymérisables par radicaux.

8. Composition d'adhésif dentaire suivant la revendication 7, dans laquelle l'anhydride d'acide est choisi dans le groupe consistant en le dianhydride d'acide butanetétracarboxylique, le dianhydride d'acide tétrahydrofurannetétracarboxylique, le dianhydride d'acide benzènetétracarboxylique et l'anhydride d'acide benzènetricarboxylique.

9. Composition d'adhésif dentaire suivant la revendication 7 ou 8, dans laquelle les dérivés d'alcools polymérisables par radicaux sont choisis dans le groupe consistant en l'acrylate d'hydroxyéthyle, l'acrylate d'hydroxypropyle, le diacrylate de glycérol, le triacrylate de pentaérythritol, le pentaacrylate de dipentaérythritol, l'acrylate d'hexanediol, l'acrylate de poly(oxyde d'éthylène), le méthacrylate d'hydroxyéthyle, le méthacrylate d'hydroxypropyle et le diméthacrylate de glycérol.

10. Composition d'adhésif dentaire suivant l'une quelconque des revendications précédentes, dans laquelle le photoinitiateur comprend une alpha-dicétone.

11. Composition suivant la revendication 10, dans laquelle ledit catalyseur comprend de la camphoquinone.

12. Composition suivant l'une quelconque des revendications précédentes, qui est emballée dans un récipient en polyéthylène.

13. Composition suivant la revendication 1, dans laquelle la charge est une nanocharge.

14. Composition suivant l'une quelconque des revendications précédentes, la composition d'adhésif présentant une adhérence à la dentine d'au moins 18 MPa.

15. Composition suivant l'une quelconque des revendications précédentes, qui est une formulation à un composant.

16. Composition suivant l'une quelconque des revendications précédentes, dans laquelle le monomère ou prépolymère polymérisable contient un groupe fonctionnel hydrolysable dans des conditions acides dans un milieu aqueux.
